Europäisches Patentamt

European Patent Office

Office européen des brevets

(11) Veröffentlichungsnummer: **0 288 892 B1**

(19)

(12) **EUROPÄISCHE PATENTSCHRIFT**

(45) Veröffentlichungstag der Patentschrift: **03.06.92**

(21) Anmeldenummer: **88106342.4**

(22) Anmeldetag: **21.04.88**

(51) Int. Cl.⁵: **C07C 209/78**, C07C 211/26

(54) **Verfahren zur Herstellung von mehrkernigen aromatischen Polyaminen.**

(30) Priorität: **01.05.87 DE 3714606**

(43) Veröffentlichungstag der Anmeldung:
**02.11.88 Patentblatt 88/44**

(45) Bekanntmachung des Hinweises auf die
Patenterteilung:
**03.06.92 Patentblatt 92/23**

(84) Benannte Vertragsstaaten:
**BE DE ES FR GB IT NL**

(56) Entgegenhaltungen:
**EP-A- 0 014 927**
**DE-A- 2 343 658**
**DE-A- 2 500 574**
**DE-A- 2 528 694**

(73) Patentinhaber: **BAYER AG**

**W-5090 Leverkusen 1 Bayerwerk(DE)**

(72) Erfinder: **Knöfel, Hartmut, Dr.**
**Dülmener Weg 21**
**W-5068 Odenthal 3(DE)**
Erfinder: **Brockelt, Michael**
**Neuenhauser Weg 1**
**W-5060 Bergisch Gladbach 2(DE)**
Erfinder: **Petinaux, Marcel, Dr.**
**Bethelstrasse 23**
**W-4150 Krefeld 1(DE)**
Erfinder: **Uchdorf, Rudolf, Di.**
**Flensburger Zeile 1 H**
**W-4150 Krefeld 11(DE)**

**Beschreibung**

Die Erfindung betrifft eine verbesserte Ausführrungsform des Verfahrens der Herstellung von mehrkernigen aromatischen Polyaminen durch Kondensation von Anilin mit Formaldehyd in Gegenwart von Wasser und sauren Katalysatoren und Aufarbeitung des Reaktionsgemisches durch Extraktion mit einem hydrophoben Lösungsmittel unter Wiederverwendung des in der wäßrigen Phase der Extraktion anfallenden Säurekatalysators.

Es ist bereits bekannt, bei der Herstellung von mehrkernigen aromatischen Polyaminen durch Kondensation von Anilin mit Formaldehyd in Gegenwart von Wasser und sauren Katalysatoren das anfallende wäßrige Reaktionsgemisch durch Extraktion mit einem hydrophoben Lösungsmittel aufzuarbeiten und den bei der Extraktion in der wäßrigen Phase anfallenden Säurekatalysators wiederzuverwenden (vgl. z.B. DE-OS 2 238 920, DE-OS 2 343 658, DE-OS 2 356 828, DE-OS 2 500 573, DE-OS 2 500 574 oder DE-OS 2 528 694).

Der wesentliche Vorteil der Verfahren dieser Vorveröffentlichungen ist darin zu sehen, daß auf eine Neutralisation des Katalysators verzichtet werden kann, da er bei der extraktiven Aufarbeitung des sauren Reaktionsgemischs in der wäßrigen Phase anfällt, die als solche an den Prozeßanfang zurückgeführt und wiederverwendet wird. Außerdem gestatten bestimmte Varianten dieses bekannten Prinzips, wie sie beispielsweise in DE-OS 2 500 574 oder 2 528 694 beschrieben sind, die gezielte Herstellung von Polyamingemischen mit wahlweise erhöhtem oder erniedrigtem Gehalt an 2,4′-Isomeren. Im übrigen entsprechen die Verfahrensprodukte der Vorveröffentlichungen bezüglich ihrer Eignung als Vorprodukt zur Herstellung von Polyisocyanaten den klassischen Polyaminen der Diphenylmethanreihe, die unter Neutralisation des eingesetzten Säurekatalystors hergestellt worden sind. Dies bedeutet, daß das Eigenschaftniveau der aus derartigen Polyisocyanatgemischen der Diphenylmethanreihe hergestellten Polyurethanschaumstoffe in beiden Fällen annähernd das gleiche ist. Im übrigen ist es als Nachteil der Verfahren der genannten Vorveröffentlichungen anzusehen, daß zur Aufarbeitung der Verfahrensprodukte durch Extraktion ausschließlich für diesen Zweck beträchtliche Mengen an hydrophoben Lösungsmittelnund Anilin eingesetzt werden müssen, was selbstverständlich auf einen beträchtlichen Destillationsaufwand und damit Energieverbrauch bei der destillativen Aufarbeitung der organischen Phase hinausläuft.

Es war die der Erfindung zugrundeliegende Aufgabe, ein neues, verbessertes Verfahren zur Herstellung von mehrkernigen aromatischen Polyaminen aus Anilin und Formaldehyd in Gegenwart von sauren Katalysatoren zur Verfügung zu stellen, welches die Vorteile der bekannten Verfahren des Standes der Technik in sich vereinigt und darüber hinaus die Herstellung von qualitativ verbesserten Produkten bei gleichzeitig reduziertem Destillationsaufwand und damit Energieverbrauch gestattet.

Diese Aufgabe konnte mit dem nachstehend näher beschriebenen erfindungsgemäßen Verfahren gelöst werden.

Beim erfindungsgemäßen Verfahren wird der eingesetzte Säurekatalysator ebenso wie bei den Verfahren des Standes der Technik nicht durch Neutralisation vernichtet, sondern durch Extraktion der Verfahrensprodukte mit Gemischen aus hydrophobem Lösungsmittel und Anilin erhalten und recyclisierbar gemacht, im Unterschied zu den Verfahren des Standes der Technik werden jedoch die bei der Extraktion verwendeten Gemische bestehend aus hydrophobem Lösungsmittel und Anilin in der bei der destillativen Aufarbeitung anfallenden Zusammensetzung nicht nur bei der Extraktion sondern teilweise auch bereits während der Umlagerungs- und/oder Kondensationsreaktion eingesetzt und damit zweifach genutzt, woraus sich ohne zusätzlichen Aufwandung beträchtliche Vorteile für das Verfahren und die resultierenden Verfahrensprodukte ergeben.

Diese Vorteile sind folgende:
- der technische Ablauf des Reaktionsgeschehens wird wesentlich verbessert durch Vermeidung von Zuständen erschwerter Rührbarkeit oder Förderfähigkeit des Reaktionsgemischs, insbesondere bei niedrigen Temperaturstufen und/oder niedrigen Anilin-Formaldehydkondensationsverhältnissen.
- Die Ausbeute an Diaminodiphenylmethan kann erhöht oder aber das Volumen der wäßrigen, den Katalysator enthaltenden Phase und damit der apparative Aufwand der Extraktionsstufe bei unveränderter Ausbeute an Diaminodiphenylmethan erniedrigt werden.
- Die Menge an Extraktionsmittel und damit die Destillationsleistung bei der Aufarbeitung der organischen Phasen kann bei gleicher Leistung erniedrigt werden.
- Die resultierenden Produkte weisen einen erhöhten Gehalt an 2,4′-Diaminodiphenylmethan bei einem gleichzeitig niedrigen Gehalt an 2,2′-Diaminodiphenylmethan auf.
- Die aus den erfindungsgemäßen Verfahrensprodukten hergestellten Polyisocyanate eignen sich hervorragend zur Herstellung von Polyurethanschaumstoffen einer geringen Eigenfarbe.

Gegenstand der Erfindung ist ein Verfahren zur Herstellung von mehrkernigen aromatischen Polyaminen durch Umsetzung von Anilin mit Formaldehyd in Gegenwart von Wasser und sauren Katalysatoren in einer ein- oder zweistufigen Reaktion innerhalb des Temperturbereichs von 0 bis 180°C, gegebenenfalls unter Vorschaltung einer Aminal-Vorstufe, in welcher in Abwesenheit von Säurekatalysator die Bildung von N,N′-disubstituiertem Aminal stattfindet, welches dann ein- oder mehrstufig in Gegenwart von Säurekatalysator innerhalb des Temperaturbereichs von 0 bis 180°C in das gewünschte Endprodukt überführt wird, Aufarbeitung des resultierenden Reaktionsgemischs durch Extraktion mit einem Anilin-haltigen hydrophoben Lösungsmittel, destillative Auftrennung der hierbei anfallenden organischen Phase in (i) ein aus Anilin-haltigem Lösungsmittel bestehendes Destillat, welches, gegebenenfalls nach Zugabe von Frischanilin erneut bei der Extraktion eingesetzt wird und (ii) einen im wesentlichen aus Verfahrensprodukt bestehenden Destillationsrückstand und Rückführung der bei der Extraktion anfallenden, den Säurekatalysator enthaltenden wäßrigen Phase unter Wiederverwendung des in ihr enthaltenen Katalysators, unter Entfernung des bei der Kondensationsreaktion entstehenden Kondensationswassers und des in das System mit der wäßrigen Lösung des Formaldehyds eingebrachten Wassers in einem der Aminalvorstufe nachgeschalteten und der ersten Reaktionsstufe vorgeschalteten Wasserabscheider und/oder in einem der Extraktionsstufe nachgeschalteten und der ersten Reaktionsstufe vorgeschalteten Verdampfer, dadurch gekennzeichnet, daß man

a) den in Form einer wäßrigen Lösung zum Einsatz gelangenden Formaldehyd in einer Aminal-Vorstue (3) mit einem Anilin-haltigen hydrophoben Lösungsmittel und/oder in der ersten Reaktionsstufe (5) mit einem Anilin-haltigen hydrophoben Lösungsmittel und der recyclisierten, den Katalystor in Form von Aminsalzen enthaltenden wäßrigen Phase durch Vermischen zur Reaktion bringt,

b) das so erhaltene zweiphasige Reaktionsgemisch nach Beendigung der Reaktion in einem der Extraktionsstufe (8) vorgeschalteten Phasenscheider (7) in eine wäßrige Phase und eine organische Phase auftrennt,

c) die im Phasenscheider anfallende organische Phase separat oder gemeinsam mit der, die Extraktionsstufe verlassenden organischen Phase destillativ in (i) ein aus Anilin-haltigem Lösungsmittel bestehendes Destillat bzw. zwei aus Anilin-haltigem Lösungsmittel bestehende Destillate und (ii) einen oder zwei im wesentlichen aus Verfahrensprodukt bestehende Destillationsrückstände auftrennt, und

d) aus dem Destillat gemäß c) oder den Destillaten gemäß c) zwei Teilströme herstellt, wobei diese beiden Teilströme auch mit den beiden Destillaten gemäß c) identisch sein können, einen der Teilströme mit dem wäßrigen Formaldehyd in der Aminal-Vorstufe oder, bei Abwesenheit einer Aminalvorstufe, in der ersten Reaktionsstufe mit dem wäßrigen Formaldehyd und der wäßrigen, den sauren Katalysator enthaltenden Phase vermischt und den anderen Teilstrom in der Extraktionsstufe zur Extraktion des Verfahrensprodukts aus der wäßrigen Phase des Reaktionsgemisches einsetzt.

Ausgangsmaterialien für das erfindungsgemäße Verfahren sind Anilin und Formaldehyd. Der Formaldehyd wird vorzugsweise in Form einer wäßrigen Lösung mit einem Formaldehydgehalt von 20 bis 50 Gew.-% eingesetzt.

Bei den zum Einsatz gelangenden hydrophoben Lösungsmitteln handelt es sich um inerte Lösungsmittel des Siedepunktbereichs 30-250°C, vorzugsweise 80-200°C wie beispielsweise Chlorbenzol, Dichlorbenzole, Benzol, Toluol, Xylol, Dichlorethan, Chloroform oder Tetrachlorkohlenstoff. Vorzugsweise werden Xylole, d.h. technische Xylolgemische und insbesondere o-Xylol als hydrophobes Lösungsmittel verwendet.

Bei dem Säurekatalysator handelt es sich um wasserlösliche Säuren mit einem unter 2,5, vorzugsweise unter 1,5 liegendem pKa-Wert. Beispiele hierfür sind Salzsäure, Bromwaserstoffsäure, Schwefelsäure, Trifluoressigsäure, Methansulufonsäure oder Phosphorsäure. Bevorzugt einzusetzender Katalysator ist Salzsäure. Die genannten Säuren können auch im Gemisch mit sauren oder neutralen Salzen derartiger Säuren, wie z.B. den entsprechenden Ammoniumsalzen oder auch den entsprechenden Alkalisalzen eingesetzt werden; die Mitverwendung derartiger Salze ist jedoch weniger bevorzugt. Die genannten Säuren liegen in dem erfindungsgemäßen Kreislaufsystem in Form der entsprechenden Ammoniumsalze der im wäßrigen Kreislauf befindlichen Basen vor.

Das erfindungsgemäße Verfahren kann sowohl ein- als auch zweistufig und sowohl unter Vorschaltung einer Aminal-Vorstufe als auch ohne eine derartige Aminal-Vorstufe durchgeführt werden, wobei jedoch einschränkend hinzugefügt werden muß, daß bei einstufiger Reaktionsführung die Vorschaltung einer Aminal-Vorstufe auf jeden Fall angezeigt ist. Unter "einstufiger Reaktionsführung" ist hierbei eine Verfahrensvariante zu verstehen, gemäß welcher das Aminal nach Hinzufügung des Säuekatalysators innerhalb eines kurzen Zeitraums von weniger als 10 Minuten, vorzugsweise von weniger als 5 Minuten auf ein erhöhte Temperatur von 60 bis 180, vorzugsweise 80 bis 150°C erhitzt wird, um dort in das Endprodukt umlagert zu werden, bzw. bei welcher das Aminal direkt mit der auf erhöhtem Temperaturniveau von 60-180°C, vorzugsweise 80 bis 150°C befindlichen und im Kreis geführten wäßrigen Katalystorphase vermischt und das Gemisch dann gegebenenfalls bis zur angestrebten Endtemperatur erhitzt wird. Unter "zweistufiger Reaktionsführung" ist eine Ausführungsform zu verstehen, gemäß welcher das Aminal nach Zugabe des Säurekatalysators oder das Reaktionsgemisch aus Anilin, Formaldehyd und Säurekatalysator zunächst während eines Zeitraums von 10 bis 90, vorzugweise 30 bis 60 Minuten in einer ersten Reaktionsstufe bei 0 bis 60°C, vorzugsweise 30 bis 60°C und anschließend während eines Zeitraums von 30 bis 180, vorzugsweise 60 bis 120 Minuten in einer zweiten Reaktionsstufe bei 60 bis 180°C, vorzugsweise 60 bis 150°C und insbesondere 100 bis 150°C gehalten wird. Bei dieser bevorzugten Verfahrensvariante einer zweistufigen Reaktionsführung erfolgt in der ersten Reaktionsstufe vor allem eine Umlagerung des Aminals oder eine Kondensation der Ausgangsmaterialien zu N-Benzylanilin, welches in der zweiten Reaktionsstufe bei erhöhter Temperatur zum kernsubstituierten Endprodukt umgelagert wird. Gemäß einer besonderen Ausführungsform mit zweistufiger Reaktionsführung - ohne oder vorzugsweise mit Aminalvorstufe - wird die erste Reaktionsstufe nur mit einem Teilstrom der wäßrigen Katalysatorphase, im allgemeinen < 50 %, vorzugsweise < 15 %, durchgeführt. Nach beendeter erster Reaktionsstufe und vor Beendigung der zweiten Reaktionsstufe erfolgt die Vervollständigung der Reaktion in Gegenwart der gesamten Katalysatorphase. Diese Ausführungsform ist besonders geeignet zur Gewinnung von Verfahrensendprodukten mit einem erhöhten Gehalt an ortho-Isomeren, insbesondere 2,4'-Diaminodiphenylmethan bei relativ geringem Anfall an 2,2'-Isomeren. Das Verfahren kann sowohl kontinuierlich als auch diskontinuierlich durchgeführt werden; die angegebenen Zeiträume beziehen sich bei kontinuierlicher Fahrweise auf die mittlere Verweilzeit des Reaktionsgemisches in den einzelnen Stufen. Im Falle der Vorschaltung einer Aminal-Vorstufe beträgt die (mittlere) Verweilzeit der Ausgangsmaterialien in dieser Stufe im allgemeinen 10 bis 60, vorzugsweise 15 bis 40 Minuten. Die Temperatur in der Aminal-Vorstufe liegt im allgemeinen bei 0 bis 60°C, vorzugsweise 20 bis 40°C. Das Verfahren wird in allen Stufen vorzugsweise unter dem Eigendruck des Systems und vorzugsweise in einer Intergasatmosphäre (Stickstoff) durchgeführt.

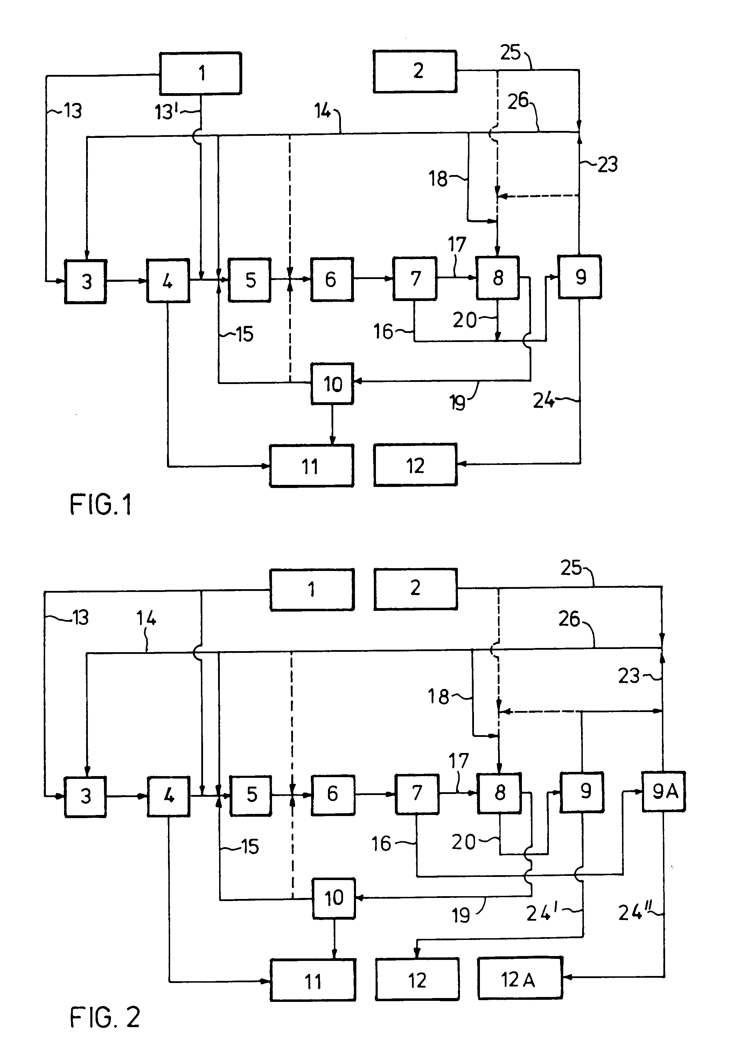

Die in den Figuren 1 und 2 dargestellten Fließdiagramme dienen der weiteren Erläuterung des erfindungsgemäßen Verfahrens. In diesen Abbildungen bedeuten:

(1)     einen Tank für wäßrige Formaldehydlösung

(2)     einen Tank für Anilin

(3)     einen Kondensationsreaktor (Aminal-Vorstufe)

(4)     einen Wasserabscheider

(5)     die erste Reaktionsstufe

(6)     die zweite Reaktionsstufe

(7)     einen Phasenscheider

(8)     die Extraktionsstufe

(9)     eine Destillationsstufe

(9A)     eine weitere Destillationsstufe

(10)     einen Wasserverdampfer

(11)     einen Tank für Abwasser

(12)     einen Tank für Verfahrensprodukt und

(12A)     einen weiteren Tank für Verfahrensprodukt.

Die Ziffern (13) bis (20) und (23) bis (26) bezeichnen die Mengenströme, auf die in den Beispielen Bezug genommen wird.

Im Falle der genannten einstufigen Reaktionsführung werden die Reaktionsstufe (5) und (6) zu einer einzigen Reaktionsstufe zusammengefaßt. Sowohl die erste als auch die zweite Reaktionsstufe können aus einem einzelnen als auch aus mehreren, in Serie geschalteten Reaktoren bestehen. Zur Aufrechterhaltung der genannten Verweilzeiten haben sind insbesondere in Serie geschaltete Rührkesselkaskaden und oder Kolonnenreaktoren bewährt. Auch die Extraktionsstufe kann aus einem oder mehreren, in Serie geschalteten Extraktoren bestehen. Hier werden vorzugsweise die üblichen Gegenstromextraktionsvorrichtungen verwendet.

Die Destillationsstufen (9) und (9A) bestehen im einfachsten Fall aus jeweils einer Destillsationskolonne, die so ausgelegt werden, daß eine weitgehende Abtrennung von hydrophobem Lösungsmittel und Anilin vom Verfahrensprodukt möglich ist.

Als ein besonderer Vorteil des erfindungsgemäßen Verfahrens ist dabei der Umstand anzusehen, daß die Auftrennung von hydrophobem Lösungsmittel und Anilin nicht erforderlich ist, da der Anilingehalt im Destillat stets unter dem für die Wiederverwendung erforderlichen Wert liegt, der vor der Wiederverwendung durch Zugabe von frischem Anilin eingestellt wird, und so der Einsatz energiesparender Mehrstufendestillationstechniken zur bewältigung der Destillationsaufgabe ermöglich wird.

Das bei der Kondensation entstehende und das mit der wäßrigen Formaldehydlösung in das System eingebrachte Wasser muß an einer geeigneten Stelle zwecks Aufrechterhaltung eines konstanten Wasservolumens aus dem System entfernt werden. Bei Vorschaltung einer Aminalvorstufe (3) geschieht diese Wasserentfernung vorzugsweise im Wasserabscheider (4) bevor das Aminal mit dem Säurekatalysator zusammengebracht wird. In Abwesenheit einer Aminalvorstufe geschieht die Entfernung des Wassers vorzugsweise in einem Wasserverdampfer (10) der der Extraktionsstufe (8) nachgeschaltet ist. Dieser Wasserverdampfer wird vorzugsweise nach dem Prinzip der Entspannungsverdampfung durch Anlegen von Vakuum betrieben. Grundsätzlich ist es jedoch auch möglich, das Wasser dem System an einer beliebigen anderen Stelle durch Destillation zu entnehmen.

Das bei der nachstehend im Detail beschriebenen Entfernung von Säurespuren aus der bzw. den organischen Phase(n) aus dem Scheider (7) bzw. aus der Extraktionsstufe (8) anfallende und an geeigneter Stelle, vorzugsweise vor der Phasentrennung in (7) oder der Extraktion der wäßrigen Phase in (8) in die Reaktionsmischung zurückgeführte Waschwasser wird ebenfalls in der Verdampferstufe (10) destillativ aus dem Kreislauf entfernt.

Die Einspeisung der wäßrigen Formaldehydlösung in das System erfolgt bei Vorschaltung einer Aminal-Vorstufe (3) im wesentlichen in dieser Aminal-Vorstufe, jedoch kann auch ein Teil des Formaldehyds nach dem Wasserabscheider (4) zugesetzt werden. Eine derartige zusätzliche Zugabe von Formaldehyd empfiehlt sich in jenen Fällen, in denen Verfahrensprodukte mit einem erhöhten Anteil an tri- und höherfunktionellen Polyaminen angebrachten Formaldehyds entspricht im allgemeinen einem Molverhältnis von Anilin : Formaldehyd in der Aminal-Vorstufe von 1,5:1 bis 25:1, vorzugsweise 1,8:1 bis 10:1. Dabei erweist sich das erfindungsgemäße Verfahren durch die Mitverwendung eines hydrophoben Lösungsmittels insbesondere bei niedrigen Anilin/Formaldehyd-Verhältnissen als technisch eindeutig überlegen im Vergleich zu analogen Verfahren ohne Mitverwendung eines hydrophoben Lösungsmittels, da bei diesen Verfahren bereits bei einem Anilin/Formaldehyd-Äquivalentverhältnis von unter 2,5:1 bei den bevorzugten Arbeitstemperaturen bis 60°C feste oder halbfeste, technisch schwer handhabbare produkte entstehen und außerdem die Abscheidung des Kondensations- und Formalin-Wassers im Folge zu geringer Dichteunterschiede Schwierigkeiten bereitet.

Falls das erfindungsgemäße Verfahren in Abwesenheit einer Aminal-Vorstufe durchgeführt wird, erfolgt die Einführung des wäßrigen Formaldehyds in das System nach der Vermischung von Anilin enthaltendem Lösungsmittel mit der ebenfalls u.a. Anilin enthaltenden wäßrigen Katalysatorphase vor der ersten Reaktionsstufe (5). In diesem Fall wird die Menge des wäßrigen Formaldhyds im allgemeinen so bemessen, daß das Molverhältnis Anilin : Formaldehyd bei 1,5:1 bis 25:1 liegt, wobei in die Berechnung des molaren Anilin/Formaldehyd-Verhältnisses sowohl das Anilin der vor dem ersten Reaktor (5) eingespeisten Anilinlösung in hydrophobem Lösungsmittel als auch das zumindest teilweise protonierte Anilin der an dieser Stelle einzuspeisen, aus dem Extraktor (8) zurückgeführten wäßrigen Phase eingeht. Hohe Anilin/Formaldehyd-Verhältnisses innerhalb der genannten breiten Bereiche sind erforderlich, um Polyamine mit hohem Diamingehalt zu erzeugen.

Das an dem Reaktionsgeschehen beteiligte Anilin setzt sich formal zusammen aus:

A1. Anilin im Gemisch mit hydrophoben Lösungsmittel wie es bei der destillativen Trennung in Destillationsstufe (9) und gegebenenfalls (9A) anfällt. Dieses Gemisch enthält stets weniger Anilin als der für die Extraktionsstufe (8) und auch für die chemische Umsetzung in der Aminalstufe (3) und/oder der ersten Reaktionsstufe (5) bevorzugt eingesetzten Mischung aus Anilin und hydrophoben Lösungsmittel entspricht. Daher muß diese Fehlmenge als

A2. Frischanilin ersetzt werden. Bei dem in der Extraktionsstufe eingesetzten Teilstrom des Destillats erfolgt diese Anilinzugabe in jedem Falle vor der Extraktionsstufe (8). Das nach dem Scheider (7) und vor der Extraktionsstufe (8) zugesetzte Anilin findet sich im wesentlichen in der wäßrigen Phase aus (8) wieder und ist Bestandteil des nachstehend unter A3. genannten Anilins. Bei den in der Kondensationsreaktion eingesetzten Teilmengen erfolgt die Frischanilinzugabe vorzugsweise ebenfalls vor der chemischen Umsetzung in (3) bzw. (5), prinzipiell ist die Zugabe auch zu einem späteren Zeitpunkt des Reaktionsablaufs möglich, beispielsweise vor der Phasenscheidung in (7). Spätestens jedoch nach der Phasentrennung muß zur wäßrigen Phase vor Eintritt in die Extraktionsstufe (8) eine den Extraktionsbedingungen äquivalente Anilinzugabe erfolgen.

A3. Einen weiteren Beitrag zum am Reaktionsgeschehen zumindest der ersten und zweiten sauren Umlagerung beteiligten Anilin liefert die wäßrige Katalysatorkreislaufphase, die die bei den Extraktionsverfahren bekannte, übliche Zusammensetzung hat und neben dem Katalysator und Wasser noch Anilin und geringe Mengen an Verfahrensprodukten enthält. Dieses Anilin setzt sich formal zusammen aus in der Reaktion im Überschuß vorhandenem und im Kreislauf geführtem Anilin und dem in der Extraktionsstufe gegen die Verfahrensprodukte ausgetauschten aus dem Extraktionsmittel stammenden Frischanilin.

Bei der bevorzugten Ausführungsform der Vorschaltung einer Aminalvorstufe setzt sich das in dieser Vorstufe einzusetzende Anilin aus A1 (Destillatteilstrom aus Anilin und hydrophoben Lösungsmittel) und A2 (Frischanilinanteil) zusammen, wobei hier das Äquivalentverhältnis von diesem Anilin zur Formaldehyd in der Aminalstufe bei 1,5:1 bis 25:1, vorzugsweise 1,8:1 bis 10:1 liegt.

Das Reaktionsprodukt der Aminalvorstufe besteht aus einer organischen Phase, die neben überschüssigem Anilin die aminalartigen Reaktionsprodukte aus Anilin und Formaldehyd eingebrachten und dem bei der Kondensationsreaktion entstandenen Wasser mögliche wasserlösliche Verunreinigungen des Formalins wie Methanol, Ameisensäure usw. und des Anilins wie Cyclohexylamin, Pyridin usw. enthält. Im Phasenscheider (4) wird die wäßrige Phase abgetrennt und die organische Phase in einer ersten Reaktionsstufe (5) mit der wäßrigen Katalysatorkreislaufphase unter Vermischen zusammengebracht, die außer dem Katalysator auch den Anilinbeitrag A3 enthält. Dadurch wird das Anilin-Formaldehydverhältnis in der zweiphasigen Reaktionsmischung an dieser Stelle des Reaktionsablaufes erhöht und damit die Zusammensetzung des Endprodukts in Richtung auf einen erhöhten Anteil an Zweikernprodukten beeinfußt.

Falls Interesse an einer möglichst hohen Ausbeute an höherkernigen Polyaminen besteht, verfährt man daher bei einer zweiten bevorzugten Ausführungsform des erfindungsgemäßen Verfahrens wie bei der ersten jedoch unter Einhaltung eines niedrigen Anilin/Formaldehyd-Verhältnisses innerhalb der genannten Bereiche vor der Aminalstufe und zusätzlich mit dem Unterschied, daß nach dem Vermischen der organischen Phase aus der Aminalvorstufe mit der wäßrigen Katalysatorkreislaufphase weiteres Formalin in (5) zugemischt wird, wobei auch die Vermischung aller drei Mengenströme, gleichzeitig in (5) erfolgen kann. Hierdurch werden im Vergleich zu der ersten Ausführungsform Verfahrensprodukte mit niedrigem Diamingehalt zugänglich, bei denen die durch die Aminalvorstufe bedingten höheren Qualitätsmerkmale anteilmäßig erhalten bleiben.

Daher ist eine dritte erfindungsgemäße Ausführungsform, bei der ganz auf die Aminalvorstufe verzichtet und der gesamte Formaldehyd in der ersten Reaktionsstufe (5) mit dem vorher vereinigten Destillatanteil aus Anilin und hydrophobem Lösungsmittel (A1) und gegebenenfalls dem Frischanilin (A2) und dem wäßrigen Katalysatorkreislauf (A3) zur Reaktion gebracht wird gegenüber den vorgenannten beiden Ausführungsformen zwar weniger bevorzugt, jedoch den bekannten einphasigen Verfahren des Standes der Technik dennoch eindeutig überlegen, da sie zu qualitativ besseren Verfahrensprodukten insbesondere im Bereich geringer Diamindiphenylmethangehalte (> 70 %) führt.

Dagegen ist eine vierte Ausführungsform des erfindungsgemäßen Verfahrens bevorzugt, bei der analog der ersten Ausführungsform gearbeitet wird mit dem Unterschied, daß die organische Phase der Aminalvorstufe (3) in der ersten Reaktionsstufe (5) zunächst mit einer Teilmenge von im allgemeinen weniger als 50 %, vorzugsweise von weniger als 15 % der wäßrigen Katalysatorkreislaufphase umgesetzt wird, vorzugsweise im angegebenen unterem Temperaturbereich und bei angemessener Verweilzeit. Die Zugabe der Restmenge erfolgt in einer weiteren Stufe nach Beendigung der ersten und vor Beendigung der zweiten Reaktionsstufe (6), wobei es nicht wesentlich ist, ob die Zugabe der Restmenge im bevorzugten unten Temperaturbreich der zweiten Reaktionsstufe (ca. 60-100 °C) oder bei erhöhter Temperatur erfolgt. Die Destillationsstufe (10) kann hierbei sowohl vor der Auftrennung der wäßrigen Phase aus (8) (gezeichnet) oder nach der Auftrennung in einem oder beiden Teilströmen (nicht gezeichnet) angeordnet sein.

Gemäß fünfter und sechster Ausführungsform des erfindungsgemäßen Verfahrens werden die Arbeitsweisen der zweiten bzw. dritten Ausführungsform mit dem in der vierten Ausführungsform ausgeführten Prinzip der Aufteilung des wäßrigen Katalysatorkreislaufs kombiniert.

Die beschriebenen Verfahrensvarianten 1-3 liefern gegenüber den bekannten einphasigen Verfahren des Standes der Technik bei vergleichbarem Gehalt an Diaminodiphenylmethan in den Reaktionsprodukten einen erhöhten Gehalt an 2,4′-Isomeren, der bei den Varianten 4-6 in weiten Grenzen gezielt weiter erhöht werden kann, bei gleichzeitig niedrigem Gehalt an 2,2′-Isomeren verglichen mit 2,4′-reichen Produkten, die nach den Verfahren des Standes der Technik im allgemeinen erhalten werden.

Die Zugabe des Anilins erfolgt vorzugsweise in Form einer 30 bis 70 gew.-%igen, insbesondere in Form einer 40 bis 70 gew.-%igen Lösung von Anilin in hydrophobem Lösungsmittel.

Bei allen genannten Varianten des erfindungsgemäßen Verfahrens ist es im Prinzip möglich, das Frischanilin und das erfindungswesentliche Lösungsmittel-Anilingemisch, welches aus der Destillationsstufe als Destillat anfällt, separat an den genannten Stellen in das System einzuführen. Eine derartige Arbeitsweise ist jedoch unwirtschaftlich und daher weniger bvorzugt.

Der Säurekatalysator wird bei Anwesenheit einer Aminal-Vorstufe bei einstufiger Fahrweise vor bzw. in der Umlagerungsstufe, bei zweistufer Fahrweise vor oder in der ersten Umlagerungsstufe (5) und gegebenenfalls, in Form eines Teilstroms, nach der ersten Umlagerungsstufe hinzugefügt. Der Katalysator kommt hierbei in Form der in der Extraktiosstufe (8) anfallenden, zurückgeführten wäßrigen Lösung der entsprechenden Ammoniumsalze zum Einsatz. Diese Lösung weist im allgemeinen neben zumindest teilseise protoniertem Anilin auch untergeordnete Mengen an zumindest teilweise protoniertem MDA auf. Der Protonierungsgrad (Prozentsatz aller Stickstoffatome, die in Form von Ammoniumgruppen vorliegen) liegt in der zurückgeführten wäßrigen Phase im allgemeinen bei 25 bis 70, vorzugsweise 40 bis 65 %. Die Konzentration an zumindest teilweise protonierten Aminen liegt in der wäßrigen Phase am Ausgang aus dem Extraktor (8) im allgemeinen bei 25 bis 60 Gew.-% und am Ausgang aus dem, gegebenenfalls zwischengeschalteten Wasserverdampfer (10) bei 30 bis 70 %. Bei allen, oben beschriebenen Ausführungsformen (Zugabe der Säure an einer bzw. an zwei unterschiedlichen Stellen) entspricht die Säuremenge im allgemeinen einem Äquivalentverhältnis von Stickstoff zu Säure am Ausgangs des Umlagerungsreaktors (einstufige Fahrweise) bzw. des zweiten Umlagerungsreaktors von 2:1 bis 20:1 vorzugsweise 3:1 bis 8:1.

Bei Anwesenheit einer Aminal-Vorstufe erfolgt vorzugsweise die Zugabe der Gesamtmenge des Lösungsmittel-Anilinemisches vor bzw. in der Aminalstufe unter Beachtung der oben gemachten Ausführungen bezüglich des Anilin/Formaldehyd-Verhältnisses, jedoch kann gegebenenfalls eine weitere Menge des Gemisches zusätzlich (i) vor oder in der Umlagerungsstufe (einstufige Fahrweise) bzw. (ii) vor oder in der ersten Umlagerungsstufe (5) und/oder vor oder in der zweiten Umlagerungsstufe (6) zugeführt werden (zweistufige Fahrweise).

Bei Abwesenheit einer Aminal-Vorstufe erfolgt die Zugabe des Lösungsmittelstroms vorzugsweise vor oder in der ersten Stufe (5), unter Beachtung der oben gemachten Ausführungen bezüglich des Anilin/Formaldehyd-Verhältnisses und gegebenenfalls einer zusätzlichen Menge des Gemisches hinter der ersten Stufe (5) und vor bzw. in der zweiten Stufe (6).

Durch den Umstand, daß das erfindungsgemäße Verfahren zweiphasig, d.h. in Gegenwart eines hydrophoben Lösungsmittels durchgeführt wird, wird erreicht, daß der Portonierungsgrad in der wäßrigen Phase stets wesentlich höher ist als es dem obengenannten Äquivalentverhältnis von Aminstickstoff zu Säure entspricht, da die Hauptmenge des nicht protonierten Amins in der Lösungsmittelphase vorliegt.

Das die Umlagerungsstufe (einstufige Fahrweise) bzw. die zweite Umlagerungsstufe (6) verlassende Reaktionsgemisch wird im Phasenscheider (7) in eine wäßrige, dem Extraktor (8) zuzuführende Phase und eine organische Phase aufgetrennt. Die hierbei anfallende organische Phase enthält bereits einen Teil des Verfahrensprodukts und wird entweder für sich allein in der Destillationsstufe (9A) oder zusammen mit der in der Extraktionsstufe (8) anfallenden organischen Phase in der Destillationskolonne (9) in Verfahrensprodukt (Destillationsrückstand) und eine Anilin-haltige Lösung (Destillat) aufgetrennt. Die in der organischen Phase aus (7) vorliegenden Verfahrensprodukte weisen stets einen höheren Gehalt an ortho-Isomeren auf als die in der wäßrigen Phase verbleibenden Verfahrensprodukte. Eine getrennte destillative Aufarbeitung ist daher dann angezeigt, wenn ein Interesse an Verfahrensprodukten mit einen erhöhten Gehalt an ortho-Isomeren besteht. Das Gewichtsverhältnis von in der organischen Phase aus (7) vorliegenden Verfahrensprodukten zu in der wäßrigen Phase aus (7) vorliegenden Verfahrensprodukten liegt im allgemeinen zwischen 0,2:1 bis 3:1 und hängt im wesentlichen von der Wasser-, Säure- und Lösungsmittelmenge des in den Phasenscheider (7) eingebrachten Reaktionsgemischs ab.

Die wäßrige, den Phasenscheider (7) verlassende Phase wird in der Extraktionsstufe (8) mit Anilin-haltigem hydrophobem Lösungsmittel extrahiert. Vorzugsweise wird hier das gleiche Lösungsmittel eingesetzt, wie es auch bereits zu Beginn bzw. während der Reaktion dem Reaktionsgemisch zugeführt worden ist. Das zur Extraktion eingesetzte Lösungsmittel-Anilingemisch weist im allgemeinen einen Anilingehalt von 30 bis 70, vorzugweise 40 bis 60 % auf.

Das Gewichtsverhältnis von organischer Phase zu wäßriger Phase am Eingang zur Extraktionsstue (8) liegt bei 0,5:1 bis 3:1, vorzugsweise 0,7:1 bis 2:1. Die Extraktiosstufe besteht im allgemeinen aus einem oder merheren in Serie gechalteten Gegenstromextraktoren. Die Extraktion wird vorzugsweise innerhalb des Temperaturbereichs von 60 bis 120, insbesondere 85 bis 100° C durchgeführt.

Die die Extraktionsstufe (8) verlassende wäßrige Phase enthält den Katalysator und wird, wie beschrieben, an den Prozeßanfang zurückgeführt. Die die Extraktionsstufe (8) verlassende organische Phase wird entweder zusammen mit der aus dem Phasenscheider (7) resultierenden organischen Phase oder separat in der Destillationsstufe (9) in Verfahrensprodukt (Destillationsrückstand) und Anilin-haltiges Lösungsmittel (Destillat) aufgetrennt. Das hierbei anfallende Destillat wird, nachdem es durch Zugabe von Frischanilin auf den ursprünglichen Anilingehalt gebracht worden ist, erneut zum Teil zur Extraktion und zum Teil am Prozeßanfang wiederverwendet.

Gemäß einer besonderen Variante der Aufarbeitung der erfindungsgemäßen Reaktionsgemische werden die den Phasenscheider (7) und/oder die Extraktionsstufe (8) verlassenden organischen Phasen vor ihrer destillativen Aufarbeitung noch einer Wasserwäschen (nicht gezeichnet) unterzogen, um geringe, mitgeschleppte Säurespuren zu entfernen. Hierzu kann beispielsweise das aus (4) bzw. (10) anfallende Wasser verwendet werden, welches nach Verlassen der vorzugsweise nach dem Prinzip einer mehrstufigen Gegenstromextraktion arbeitenden Wasserwäsche zusammen mit den ausgewaschenen Säurespuren in das Reaktionsgemisch an einer beliebigen Stelle vor der Extraktionsstufe (8) zurückgeführt wird. Besonders bevorzugt ist hierbei (i) die Rückspeisung in das Reaktionsgemisch nach beendeter zweiter Umlagerung (6) und vor Eintritt in den Phasenscheider (7) oder (ii) in die wäßrige Phase nach Verlassen des Phasenscheiders (7) und vor Eintritt in die Extraktionsstufe (8), wobei besonders bevorzugt der die Extraktionsstufe (8) verlassenden wäßrigen Phase in (10) destillativ eine Wassermenge entzogen wird, die über die Wassermenge hinaus geht, die zur Aufrechterhaltung eines konstanten Wasservolumens im Kreislauf notwendig ist und diese über die zur Konstanthaltung des Wasservolumens hinausgehende Wassermenge für die Wasserwäsche verwendet wird. Auch in Abwesenheit einer Wasserwäsche ist es oftmals von Vorteil, eine derartige, (10) entnommene Wassermenge direkt in das Reaktionsgemisch an den genannten Stellen (i) bzw. (ii) zurückzuführen.

Das Gewichtsverhältnis von eingesetztem Wasser bzw. von direkt in das Reaktionsgemisch zurückgeführtem Wasser zu der an der jeweiligen Stelle jeweils vorliegenden organischen Phase beträgt im allgemeinen 1:5 bis 1:50, vorzugsweise 1:8 bis 1:20.

Die nachfolgenden Beispiele dienen zur weiteren Erläuterung des erfindungsgemäßen Verfahrens.

Beispiel 1 (Figur 1)

In einem aus zwei hintereinandergeschalteten Rührkesseln bestehenden Reaktor (3) wird 30 %ige wäßrige Formalinlösung (Mengenstrom (13)) mit einem Anilin-Xylolgemisch (Mengenstrom 14)) bei 40°C zur Reaktion gebracht.

(13) 0,5 kg/h Formaldehyd
1,16 kg/h Wasser
(14) 3,10 kg/h Anilin
2,60 kg/h ortho-Xylol

In dem anschließenden Scheider (4) wird die untere wäßrige Phase als Abwasser bei 40°C abgetrennt.

Die obere, organische Phase wird in einem zweiten, aus drei Rührkesseln bestehenden Reaktor (5) übergeführt und dort mit dem Mengenstrom (15) vermischt.

(15) 0,1 kg/h Polyarylamin
1,83 kg/h Anilin
0,44 kg/h Chlorwasserstoff
2,64 kg/h Wasser

Die gemessenen und geregelten Temperaturen in den drei Kesseln des Reaktors (5) betragen 30°C, 40°C und 60°C.

In einem weiteren, ebenfalls aus drei Rührkesseln bestehenden Reaktor (6) werden die Temperaturstufen 100°C, 130°C und 140°C durchlaufen, die durch Aufheizen unter dem sich einstellenden Eigendruck des Reaktionsgemisches eingestellt werden.

Nach dem Abkühlen des Reaktionsgemisches auf 95°C und Entspannen auf Normaldruck und nach Zugabe des HCl-Waschwassers aus der (nicht gezeichneten), der Extraktionsstufe (8) nachgeschalteten Extraktionsstufe (8A) (Mengenstrom (22)) werden im Phasenscheider (7) die organische (Mengenstrom (16)) und die wäßrige Phase (Mengenstrom (17)) voneinander getrennt.

(16) 1,09 kg/h Polyarylamin
1,18 kg/h Anilin
2,60 kg/h ortho-Xylol
(17) 2,09 kg/h Polyarylamin
0,70 kg/h Anilin
0,45 kg/h Chlorwasserstoffsäure
4,04 kg/h Wasser

Die wäßrige Phase wird anschließend in der Extrationskolonne (8) im Gegenstrom mit einem Anilin-Xylolgemisch (Mengenstrom (18)) kontinuierlich extrahiert.

(18) 3,92 kg/h Anilin
3,28 kg/h ortho-Xylol

Die extrahierte wäßrige Phase aus (8) (Mengenstrom (19)) wird in der Destillationsstufe (10) aufkonzentriert unter Entnahme des Mengenstroms (21) (nicht gezeichnet) und anschließend als Mengenstrom (15) in den Reaktor (5) zurückgeführt.

Die resultierende organische Phase aus (8) (Mengenstrom (20)) wird mit der organischen Phase aus dem Scheider (7) (Mengenstrom (16)) vereinigt und in einer weiteren 3-5 stufig wirkenden Extraktionskolonne (8A) (nicht gezeichnet) mit dem im wesentlichen aus Wasser bestehenden Destillat der Destillationsstufe (10) (Mengenstrom (21)) (nicht gezeichnet) extrahiert.

(20) 1,99 kg/h Polyarylamin
2,81 kg/h Anilin
3,27 kg/h ortho-Xylol
(21) 1,38 kg/h Wasser

In der Waschstufe (8A) (nicht gezeichnet) wird der HCl-Gehalt der Mengenströme (16) und (20), der bei ca. 0,2-0,3 Gew.-% liegt, unter den angegebenen Bedingugnen reduziert auf < 0,01 Gew.-%.

Das HCl-haltige Waschwasser (ca. 1,5 kg/h) wird als Mengenstrom (22) (nicht gezeichnet) in das Reaktionsgemisch recyclisiert.

Die die Waschkolonne (8A) verlassende organische Phase (16) und (20) wird nach Befreiung von restlichen Säurespuren durch Neutralisation mit überschüssiger Natronlauge und Abtrennung des gebildeten Kochsalzes und der unverbrauchten Natronlauge in einer Destillationsstufe (9) destillativ augetrennt in ein Destillat (Mengenstrom (23)) und einen aus Polyarylamin bestehenden Destillationsrückstand (Mengenstrom (24)).

(23) 4,23 kg/h Anilin

5,86 kg/h ortho-Xylol (< 0,5 Gew.-% Polyarylamin)

(24) 3,00 kg/h Polyarylamin (< 0,1 Gew.-% Anilin)

Nach Zugabe von frischem Anilin (Mengenstrom (25)) aus dem Vorratstank (2) zu Mengenstrom (23) wird das so hergestellte Anilin-Xylolgemisch (Mengenstrom (26)) mengenmäßig aufgeteilt und als Mengenströme (14) und (18) wiederverwendet.

Der Destillationsrückstand (24) der Destillationsstufe (9) hat folgende Zusammensetzung:

67 Gew.-% Diaminodiphenylmethane

33 Gew.-% Mehrkern-Polyarylamine

Die Zweikernpolyarylaminfraktion hatte folgende Zusammensetzung:

0,5 Gew.-% 2,2$'$-Diaminodiphenylmethan

11,9 Gew.-% 2,4$'$-Diaminodiphenylmethan

87,6 Gew.-% 4,4$'$-Diaminodiphenylmethan

Beispiel 2 (Figur 1)

Man verfährt analog Beispiel 1, mit dem Unterschied, daß zusätzlich zu der Formalineinspeisung in Reaktor (3) (Mengenstrom (13)) eine weitere Formalinzugabe in Reaktor (5) erfolgt (Mengenstrom 13$'$ = 0,66 kg/h 30 %iges Formalin).

Entsprechend erfolgt zur Aufrechterhaltung eines konstanten Wasserhaushalts im Katalysatorkreislauf zusätzlich zu der im Scheider (4) durchgeführten Wasserabtrennung die Entnahme einer entsprechenden Wassermenge (ca. 0,6 kg/h) aus dem entsprechend vergrößerten Destillat der Verfdampferstufe (10).

(13) 0,5 kg/h Formaldehyd

1,16 kg/h Wasser

(14) 3,10 kg/h Anilin

2,60 kg/h ortho-Xylol

(15) =0,1 kg/h Polyarylamin

1,83 kg/h Anilin

0,44 kg/h Chlorwasserstoffsäure

2,69 kg/h Wasser

(13$'$)0,2 kg/h Formaldehyd

0,46 kg/h Wasser

(16) 1,11 kg/h Polyarylamin

0,83 kg/h Anilin

2,58 kg/h ortho-Xylol

(17) 2,9 kg/h Polyarylamin

0,4 kg/h Anilin

0,46 kg/h Chlorwasserstoffsäure

4,05 kg/h Wasser

(18) 5,58 kg/h Anilin

4,65 kg/h ortho-Xylol

(19) 0,1 kg/h Polyarylamin

1,82 kg/h Anilin

0,44 kg/h Chlorwasserstoffsäure

3,81 kg/h Wasser

(20) 2,86 kg/h Polyarylamin

4,16 kg/h Anilin

4,65 kg/h ortho-Xylol

(23) 3,95 kg/h Polyarylamin

4,92 kg/h Anilin

7,23 kg/h ortho-Xylol

(24) 3,95 kg/h Polyarylamin

(25) 3,66 kg/h Anilin

Der Destillationsrückstand (24) der Destillationsstufe (9) hat folgende Zusammensetzung:

52 Gew.-% Diaminodiphenylmethane

48 Gew.-% Mehrkern-Polyarylamine

Die Zweikernpolyaminfraktion hat folgende Zusammensetzung:

0,6 Gew.-% 2,2$'$-Diaminodiphenylmethan

10,6 Gew.-% 2,4$'$-Diaminodiphenylmethan

88,8 Gew.-% 4,4$'$-Diaminodiphenylmethan

Beispiel 3 (Figur 1)

Man verfährt analog Beispiel 1 in der gleichen Versuchsanlage mit der gleichen Versuchsanordnung jedoch mit folgenden geänderten Mengenströmen:

(13) 0,24 kg/h Formaldehyd

0,56 kg/h Wasser

(14) 5,74 kg/h Anilin

4,40 kg/h ortho-Xylol

(15) 0,23 kg/h Polyarylamin

3,77 kg/h Anilin

0,65 kg/h Chlorwasserstoffsäure

3,22 kg/h Wasser

(16) 0,54 kg/h Polyarylamin

5,23 kg/h Anilin

4,40 kg/h ortho-Xylol

(17) 1,04 kg/h Polyarylamin

3,01 kg/h Anilin

0,65 kg/h Chlorwasserstoffsäure

3,22 kg/h Wasser

(18) 8,26 kg/h Anilin

6,30 kg/h ortho-Xylol

(20) 0,81 kg/h Polyarylamin

7,50 kg/h Anilin

6,29 kg/h ortho-Xylol

(21) 1,15 kg/h Wasser

(23) 12,73 kg/h Anilin

10,74 kg/h ortho-Xylol

(24) 1,35 kg/h Polyarylamin

(25) 1,27 kg/h Anilin

Der Destillationsrückstand (24) der Destillationsstufe (9) hat folgende Zusammensetzung:

92 Gew.-% Diaminodiphenylmethane

8 Gew.-% Mehrkern-Polyarylamin3

Die Zweikernpolyarylaminfraktion hat folgende Zusammensetzung:

1,6 Gew.-% 2,2$'$-Diaminodiphenylmethan

21,4 Gew.-% 2,4$'$-Diaminodiphenylmethan

77,0 Gew.-% 4,4$'$-Diaminodiphenylmethan

Beispiel 4 (Figur 2)

Dieses Beispiel entspricht Beispiel 3, mit dem Unterschied, daß der organische Mengenstrom (20) in der Destillationsstufe (9) zu einem Polyarylaminteilprodukt-1 (Mengenstrom (24$'$)) und der organische Mengenstrom (16) in einer separaten Destillsationsstufe (9A) zu einem Polyarylaminteilprodukt-2 (Mengenstrom (24$''$)) aufgearbeitet wird.

Die Destillate wurden vereinigt zum Mengenstrom (23).

Die Destillationsrückstände haben die folgende Zusammensetzung:

Polyarylamin-1 (0,810 kg/h):

91,3 Gew.-% Diaminodiphenylmethane
8,7 Gew.-% Mehrkern-polyarylamine
Die Zweikernpolyarylaminfraktion hatte folgende Zusammensetzung:
0,7 Gew.-% 2,2$'$-Diaminodiphenylmethan
17,1 Gew.-% 2,4$'$-Diaminodiphenylmethan
82,2 Gew.-% 4,4$'$-Diaminodiphenylmethan

Polyarylamin-2 (0,540 kg/h):

92,7 Gew.-% Diamiodiphenylmethane
7,3 Gew.-% Mehrkern-polyarylamine
Die Zweikernpolyarylaminfraktion hat folgende Zusammensetzung:
3,1 Gew.-% 2,2$'$-Diamiondiphenylmethan
29,2 Gew.-% 2,4$'$-Diaminodiphenylmethan
67,7 Gew.-% 4,4$'$-Diaminodiphenylmethan

Beispiel 5 (Figur 1)

Man verfährt analog Beispiel 3 nur mit dem Unterschied, daß unter Umgehung des Reaktors (3) und des Scheiders (4) die Mengenströme (13) und (14) direkt in Reaktor (5) mit Mengenstrom (15) vermischt und zur Reaktion gebracht werden. Demzufolge muß anders als in Beispiel 3, wo die Abtrennung des Formalin- und Kondensationswassers im Scheider (4) erfolgt, in der Destillationsstufe (10) zur Aufrechterhaltung des Wasserhaushalts eine entsprechend vergrößerte Wassermenge (II) abdestilliert werden (Wegfall der Wasserentnahme in (4)).

Der Destillationsrückstand (24) der Destillationsstufe (9) hat folgende Zusammensetzung:
89 Gew.-% Diaminodiphenylmethane
11 Gew.-% Mehrkern-Polyaylamine
Die Zweikernpolyarylaminfraktion hat folgende Zusammensetzung:
1,5 Gew.-% 2,2$'$-Diaminodiphenylmethan

20,1 Gew.-% 2,4$'$-Diaminodiphenylmethan
78,4 Gew.-% 4,4$'$-Diaminodiphenylmethan

## Patentansprüche

1. Verfahren zur Herstellung von mehrkernigen aromatischen Polyaminen durch Umsetzung von Anilin mit Formaldehyd in Gegenwart von Wasser und sauren Katalysatoren in einer ein- oder zweistufigen Reaktion innerhalb des Temperaturbereichs von 0 bis 180°C, gegebenenfalls unter Vorschaltung einer Aminal-Vorstufe, in welcher in Abwesenheit von Säurekatalysator die Bildung von N,N$'$-disubstituiertem Aminal stattfindet, welches dann ein- oder mehrstufig in Gegenwart von Säurekatalysator innerhalb des Temperaturbereichs von 0 bis 180°C in das gewünschte Endprodukt überführt wird, Aufarbeitung des resultierenden Reaktionsgemischs durch Extraktion mit einem Anilin-haltigen hydrophoben Lösungsmittel, destillative Auftrennung der hierbei anfallenden organischen Phase in (i) ein aus Anilinhaltigem Lösungsmittel bestehendes Destillat, welches, gegebenenfalls nach Zugabe von Frischanilin erneut bei der Extraktion eingesetzt wird und (ii) einen im wesentlichen aus Verfahrensprodukt bestehenden Destillationsrückstand und Rückführung der bei der Extraktion anfallenden, den Säurekatalysator enthaltenden wäßrigen Phase unter Wiederverwendung des in ihr enthaltenen Katalysators, unter Entfernung des bei der Kondensationsreaktion entstehenden Kondensationswassers und des in das System mit der wäßrigen Lösung des Formaldehyds eingebrachten Wassers in einem der Aminalvorstufe nachgeschalteten und der ersten Reaktionsstufe vorgeschalteten Wasserabscheider und/oder in einem der Extraktionsstufe nachgeschalteten und der ersten Reaktionsstufe vorgeschalteten Verdampfer, dadurch gekennzeichnet, daß man

a) den in Form einer wäßrigen Lösung zum Einsatz gelangenden Formaldehyd in einer Aminal-Vorstufe (3) mit einem Anilin-haltigen hydrophoben Lösungsmittel und/oder in der ersten Reaktionsstufe (5) mit einem Anilin-haltigen hydrophoben Lösungsmittel und der recyclisierten, den Katalysator in Form von Aminsalzen enthaltenden wäßrigen Phase durch Vermischen zur Reaktion bringt,

b) das so erhaltene zweiphasige Reaktionsgemisch nach Beendigung der Reaktion in einem der Extraktionsstufe (8) vorgeschalteten Phasenscheider (7) in eine wäßrige Phase und eine organische Phase auftrennt,

c) die im Phasenscheider anfallende organische Phase separat oder gemeinsam mit der, die Extraktionsstufe verlassenden organischen Phase destillativ in (i) ein aus Anilinhaltigem Lösungsmittel bestehendes Destillat bzw. zwei aus Anilin-haltigem Lösungsmittel bestehende Destillate und (ii) einen oder zwei im wesentlichen aus Verfahrensprodukt bestehende Destillationsrückstände auftrennt, und

d) aus dem Destillat gemäß c) oder den Destillaten gemäß c) zwei Teilströme herstellt, wobei diese beiden Teilströme auch mit den beiden Destillaten gemäß c) identisch sein können, einen der Teilströme mit dem wäßrigen Formaldehyd in der Aminal-Vorstufe oder, bei Abwesenheit einer Aminalvorstufe, in der ersten Reaktionsstufe mit dem wäßrigen Formaldehyd und der wäßrigen, den sauren Katalysator enthaltenden Phase vermischt und den anderen Teilstrom in der Extraktionsstufe zur Extraktion des Verfahrensprodukts aus der wäßrigen Phase des Reaktionsgemisches einsetzt.

2. Verfahren gemäß Anspruch 1, dadurch gekennzeichnet, daß man die Extraktionsstufe verlassenden wäßrigen Phase destillativ eine Wassermenge entzieht, die über die Wassermenge hinausgeht, die zur Aufrechterhaltung eines konstanten Wasservolumens im Kreislauf notwendig ist und diese über die zur Konstanthaltung des Wasservolumens hinausgehende Wassermenge an einer beliebigen Stelle hinter der letzten Reaktionsstufe und vor der Extraktionsstufe in den Kreislauf zurückführt.

3. Verfahren gemäß Anspruch 1, dadurch gekennzeichnet, daß man die den Säurekatalysator enthaltende wäßrige Phase nach Verlassen der Extraktionsstufe und vor ihrer Wiederverwendung in zwei Teilströme auftrennt und den einen Teilstrom in die erste Reaktionsstufe (5) und den anderen Teilstrom nach beendeter erster und vor beendeter zweiter Reaktionsstufe (6) dem Reaktionsgemisch zusetzt.

## Claims

1. A process for the production of polynuclear aromatic polyamines by reaction of aniline with formaldehyde in the presence of water and acidic catalysts in a one- or two-stage reaction at a temperature in the range from 0 to 180° C, optionally with inclusion of a preliminary aminal stage, in which N,N'-disubstituted aminal is formed in the absence of acid catalyst and is then converted into the desired end product in one or more stages in the presence of acid catalyst at a temperature in the range from 0 to 180° C, working up of the resulting reaction mixture by extraction with an aniline-containing hydrophobic solvent, separation of the organic phase accumulating by distillation into (i) a distillate consisting of aniline-containing solvent which is reused in the extraction step, optionally after addition of fresh aniline, and (II) a distillation residue consisting essentially of process product and recycling of the aqueous phase containing the acid catalyst which accumulates during the extraction step, so that the catalyst present therein may be reused, with removal of the water of condensation formed during the condensation reaction and the water introduced into the system with the aqueous solution of the formaldehyde in a water separator following the preliminary aminal stage and preceding the first reaction stage and/or in an evaporator following the extraction stage and preceding the first reaction stage, characterized in that

a) the formaldehyde used in the form of an aqueous solution is reacted by mixing in a preliminary aminal stage (3) with an aniline-containing hydrophobic solvent and/or in the first reaction stage (5) with an aniline-containing hydrophobic solvent and the recycled aqueous phase containing the catalyst in the form of amine salts,

b) on completion of the reaction, the two-phase reaction mixture obtained is separated into an aqueous phase and an organic phase in a phase separator (7) preceding the extraction stage (8),

c) the organic phase accumulating in the phase separator is separated by distillation - either separately or together with the organic phase leaving the extraction stage - into (i) a distillate consisting of aniline-containing solvent or two distillates consisting of aniline-containing solvent and (ii) one or two distillation residues consisting essentially of process product,

d) two component streams are formed from the distillate according to c) or from the distillates according to c) and may even be identical with the distillates according to c), one of component streams is mixed with the aqueous formaldehyde in the preliminary aminal stage or, in the absence of a preliminary aminal stage, in the first reaction stage with the aqueous formaldehyde and the aqueous phase containing the acidic catalyst and the other component stream is used in the extraction stage for extraction of the process product from the aqueous phase of the reaction mixture.

**2.** A process as claimed in claim 1, characterized in that water is removed by distillation from the aqueous phase leaving the extraction stage in a quantity exceeding the quantity of water required to maintain a constant volume of water in the circuit and this quantity of water exceeding that required to keep the water volume constant is returned to the circuit at any point after the last reaction stage and before the extraction stage.

**3.** A process as claimed in claim 1, characterized in that, after leaving the extraction stage and before being reused, the aqueous phase containing the acid catalyst is separated into two component streams and one component stream is introduced into the reaction stage (5) while the other component stream is added to the reaction mixture after completion of the first and before completion of the second reaction stage (6).

**Revendications**

**1.** Procédé de production de polyamines aromatiques contenant plusieurs noyaux, par réaction d'aniline avec du formaldéhyde en présence d'eau et de catalyseurs acides dans une réaction à une ou deux étapes dans la plage de températures de 0 à 180°C, le cas échéant avec intercalation d'un étage préalable d'aminal, dans lequel a lieu en l'absence de catalyseur acide la formation d'un aminal N,N'-disubstitué qui est ensuite transformé en une ou plusieurs étapes en présence d'un catalyseur acide dans la plage de températures de 0 à 180°C en le produit final désiré, traitement du mélange réactionnel résultant par extraction avec un solvant hydrophobe contenant de l'aniline, fractionnement par distillation de la phase organique ainsi obtenue (i) en un distillat constitué d'un solvant contenant de l'aniline, qui est réutilisé lors de l'extraction le cas échéant après addition d'aniline fraîche et (ii) en un résidu de distillation principalement formé du produit du procédé et recyclage de la phase aqueuse obtenue lors de l'extraction, contenant le catalyseur acide, avec réutilisation du catalyseur contenu dans cette phase, avec élimination de l'eau de condensation produite lors de la réaction de condensation et de l'eau introduite dans le système avec la solution aqueuse de formaldéhyde dans un séparateur d'eau en aval de l'étage préalable d'aminal et en amont du premier étage réactionnel et/ou dans un évaporateur en aval de l'étage d'extraction et en amont du premier étage réactionnel, caractérisé en ce que

a) on fait réagir le formaldéhyde utilisé sous forme d'une solution aqueuse dans un étage préalable d'aminal (3) avec un solvant hydrophobe contenant de l'aniline et/ou dans le premier étage de réaction (5) avec un solvant hydrophobe contenant de l'aniline et on introduit le catalyseur en vue de la réaction par mélange sous forme de phase aqueuse contenant des sels d'amine,

b) on fractionne le mélange réactionnel de deux phases ainsi obtenu, une fois la réaction terminée, dans un séparateur de phases (7) monté en amont de l'étage d'extraction (8) en une phase aqueuse et en une phase organique,

c) on fractionne la phase organique présente dans le séparateur de phases séparément ou conjointement avec la phase organique sortant de l'étage d'extraction par distillation (i) en un distillat constitué de solvant contenant de l'aniline ou en deux distillats constitués de solvant contenant de l'aniline et (ii) en un ou deux résidus de distillation principalement constitués du produit du procédé et

d) on prépare à partir du distillat selon c) ou des distillats selon c) deux courants partiels qui peuvent aussi être identiques aux deux distillats selon c), on mélange l'un des courants partiels avec le formaldéhyde aqueux dans l'étage préalable d'aminal ou, en l'absence d'un étage préalable d'aminal, dans le premier étage réactionnel avec le formaldéhyde aqueux et la phase aqueuse contenant le catalyseur acide et on utilise l'autre courant partiel dans l'étage d'extraction pour extraire le produit du procédé de la phase aqueuse du mélange réactionnel.

**2.** Procédé suivant la revendication 1, caractérisé en ce qu'on chasse par distillation de la phase aqueuse sortant de l'étage d'extraction une quantité d'eau qui est supérieure à la quantité d'eau nécessaire pour entretenir un volume constant d'eau dans le circuit et on la recycle dans le circuit en plus de la quantité d'eau suffisante pour maintenir le volume d'eau constant à un endroit quelconque en arrière du dernier étage de réaction et en avant de l'étage d'extraction.

**3.** Procédé suivant la revendication 1, caractérisé en ce qu'on fractionne la phase aqueuse contenant le catalyseur acide après sa sortie de l'étage d'extraction et avant sa réutilisation en deux courants partiels et on envoie un courant partiel dans le premier étage de réaction (5) et on ajoute l'autre courant partiel au mélange réactionnel après la fin de la première étape de réaction et avant la fin de la seconde étape de réaction (6).

FIG.1

FIG. 2

14